# EUROPEAN PATENT SPECIFICATION

(11) **EP 4 390 374 B1**
(45) Date of publication and mention of the grant of the patent: **25.03.2026**
(21) Application number: 23189120.1
(22) Date of filing: 02.08.2023
(51) Int. Cl.: G01N 21/31, G01N 33/02, G01J 3/28, G01N 21/64, G01N 21/84, G01N 21/63

(54) **QUALITY INSPECTION METHOD OF INSPECTING SMALL FRUITS AND BERRIES PRODUCT**
VERFAHREN ZUR QUALITÄTSPRÜFUNG VON KLEINFRÜCHTE- UND BEERENPRODUKTEN
PROCÉDÉ D'INSPECTION DE LA QUALITÉ DES PETITS FRUITS ET DES BAIES

(30) Priority: 23.12.2022 IT 202200026829
(43) Date of publication of application: 26.06.2024
(73) Proprietor: Sant'Orsola S.C.A., 38057 Ciré di Pergine Valsugana (Trento) (IT)
(72) Inventor: OSLER, Gilberto, 38057 Canezza di Pergine Valsugana (Trento) (IT); PERENZONI, Severino, 38121 Trento (IT)
(74) Representative: Rapisardi, Mariacristina

(56) References cited:
- US-A1- 2020 132 651
- US-A1- 2022 215 554
- BONET ISIS ET AL: "Oil Palm Detection via Deep Transfer Learning", 2020 IEEE CONGRESS ON EVOLUTIONARY COMPUTATION (CEC), IEEE, 19 July 2020 (2020-07-19), pages 1 - 8, XP033820116, DOI: 10.1109/CEC48606.2020.9185838

## Description

The present invention relates to a method of quality inspection of a small fruits and berries product.

Machines for quality inspection of fruit and vegetables based on multispectral optical reconstruction have long been on the market.

Some well-known machines, particularly sizing machines, have a singulator that separates and sorts individual units of bulk product into rows that are sequentially passed through a multispectral optical reconstruction station.

Such inspection machines, while highly efficient in product characterisation, suffer from drawbacks related to their complexity and bulkiness, particularly the horizontal clearance required to move bulk product units away from each other.

These inspection machines also suffer from the drawback that they are not recommended for the inspection of very delicate fruit and vegetables, particularly small fruits and berries, as they require rather invasive handling of the products, which could consequently spoil as a result of the inspection.

US2022215554 A1 discloses a method and system for performing characterization of one or more materials; US 2020/0132651 A1 relates to systems and methods for monitoring agricultural products; scientific publication BONET ISIS et al. is proposing a system using deep learning algorithms and the transfer learning approach to detect oil palm units in multispectral photographs taken with unmanned aerial vehicles.

The technical task proposed by the present invention is, therefore, to realise a method of quality inspection of a small fruits and berries product that eliminates the technical drawbacks present in the known technique.

Within the scope of this technical task, one aim of the invention is to realise a particularly effective and recommendable method of quality inspection of a small fruits and berries product for the inspection of small fruits, particularly small delicate fruits such as strawberries, blueberries, blackberries, raspberries, currants, grapes.

Another purpose of the invention is to realise a method of quality inspection of a small fruits and berries product that can be performed in confined spaces with space-saving instrumentation. The technical task, as well as these and other purposes, according to the present invention are achieved by a quality inspection method of inspecting a small fruits and berries product , according to Claim 1.

This container is lit from above.

Preferably such optical reconstruction means are configured for chlorophyll fluorescence imaging.

Preferably, such optical reconstruction means are configured for imaging in a plurality of selected bands in the visible and near-infrared spectrum.

Preferably such optical reconstruction means have selectable filters for band acquisition.

Preferably said acquisition with said multispectral sensor means is performed statically with said container stationary at a station.

Preferably, said station is positioned at a base of a camera obscura, and said multispectral sensor means and said multispectral illuminating means are positioned at a ceiling of said camera obscura.

Preferably, said container is supported by an indexed conveyor belt configured to station said container at said station.

Preferably, these quality indices include a maturity index of the single loose units of product.

Preferably, these quality indices include a hardness index of the single loose units of product.

Further features and advantages of the invention will become more apparent from the description

Preferably said acquisition with said multispectral sensor means is performed statically with said container stationary at a station.

Preferably, said station is positioned at a base of a camera obscura, and said multispectral sensor means and said multispectral illuminating means are positioned at a ceiling of said camera obscura.

Preferably, said container is supported by an indexed conveyor belt configured to station said container at said station.

Preferably, these quality indices include a maturity index of the single loose units of product.

Preferably, these quality indices include a hardness index of the single loose units of product.

Further features and advantages of the invention will become more apparent from the description of a preferred but not exclusive form of execution of the method of inspection according to the invention, illustrated by way of illustration and not limitation in the accompanying drawings, in which
Figure 1 shows a possible layout of the inspection machine with the container positioned in a fixed housing;

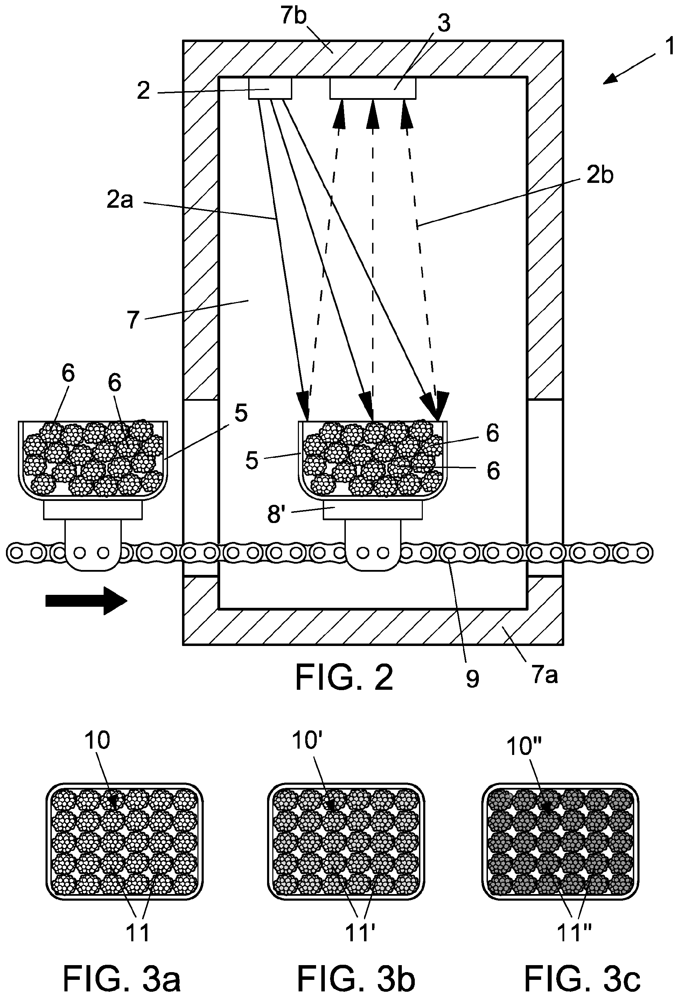

Figure 2 shows a possible layout of the inspection machine with the container positioned in a movable housing;
Figures 3a, 3b, 3c show segmented multispectral images of the product mass present in the container where the segmentation highlights the contours of the single loose units of product that form the upper layer of the product mass.

With reference to the above-mentioned figures, a method of quality inspection of a small fruits and berries product is shown, which finds application in the field of inspection of small fruits and berries, particularly cherries, strawberries, blueberries, blackberries, raspberries, currants, grapes.

The inspection method is performed with the aid of an inspection machine generically referred to by numerical reference 1, which has optical reconstruction means including multispectral illuminating means 2, e.g. LED emitters, and multispectral sensor means 3, e.g. a multispectral camera.

The inspection machine 1 has a station 4 where a container 5 filled with loose units 6 of the fruit and vegetable product can be positioned.

Container 5 is of the basket type with an open top.

Station 4 is located inside inspection chamber 7 of inspection machine 1.

The inspection chamber 7 is preferably darkened during the activation of the optical reconstruction means.

The darkening of the inspection chamber 7 is made possible, for example, by the closing of one or more of the access doors to the inspection chamber 7 required for the entry and exit of the container 5 into/from the station 4.

In the case illustrated, the inspection chamber 7 has a base 7a at which station 4 is provided and a ceiling 7b at which the optical reconstruction means are provided, particularly the multispectral sensor means 3 and the multispectral illuminator means 2.

The illumination of the product contained in container 5 is provided from above, as there are no barriers, other than air, between the multispectral illuminating means and the contents of the top-open container 5, nor between the multispectral sensor means and the contents of the top-open container 5.

The container 5 is transparent to the wavelengths emitted by the multispectral illuminating means 2 and the product contained in the container 5 thus illuminated, so that in other solutions the illumination of the product contained in the container 5 can also be performed from the side and/or from below.

Acquisition with the multispectral media sensors is performed statically with container 5 stationary at station 4.

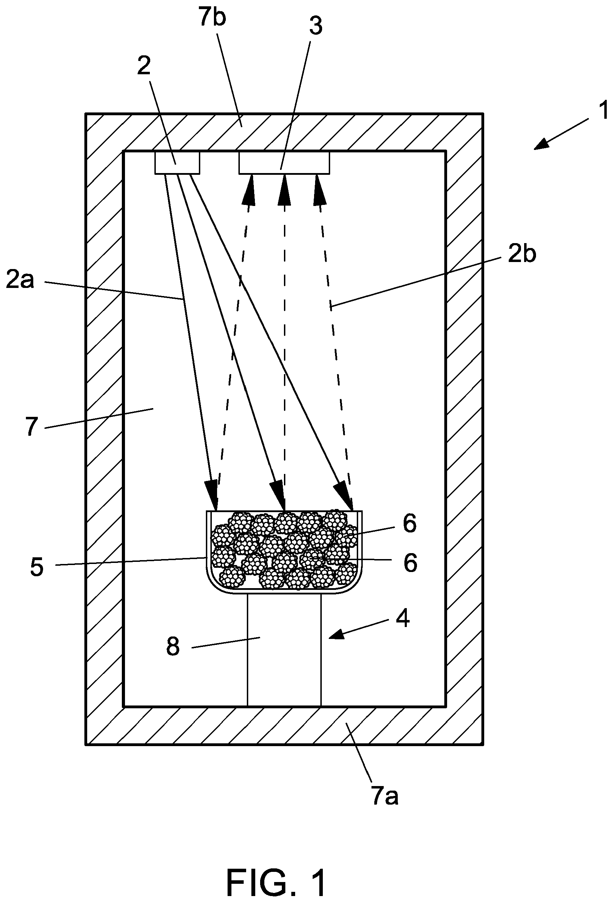

Container 5 can be supported at station 4 by a fixed housing 8, as illustrated in the solution in figure 1, or by a housing 8' supported by an indexed conveyor 9 configured to station container 5 at station 4.

The optical reconstruction means may include selectable filters for the acquisition of only one or only some bands emitted by the product contained in the container 5 illuminated by the multispectral illuminator means 2.

Depending on the application, a different filter can be interposed between the multispectral sensor means 3 and the product contained in the container 5.

A filter can be used, for example, to eliminate the image of container 5 and allow only the image of the product it contains to be captured.

A filter can also be used to identify the presence of a specific type of product defect.

Optical reconstruction means are preferably configured for imaging in a plurality of selected bands in the visible and near-infrared spectrum.

Specifically, the optical reconstruction means are configured for imaging in the following bands: 450 nm (blue), 520 nm (green), 590 nm (yellow), 630 nm (orange), 660 nm (red), 730 nm (red), 850 nm (near infrared), 940 nm (near infrared).

**In** addition, optical reconstruction means are configured for chlorophyll fluorescence imaging.

The inspection method basically involves the following sequence of steps.

**In** container 5, single loose units 6 of fruit and vegetables are stored.

The container 5 filled in this way is taken to station 4 of the inspection machine 1.

The product mass contained in container 1 is illuminated by multispectral illuminating means 2 that emit a sequence of light radiations 2a having different bandwidths.

The product mass thus illuminated in turn reflects and/or emits light radiation 2b which, when acquired by the multispectral sensor means 3, forms multispectral images 10, 10', 10" of the product mass.

Multispectral 10, 10', 10" images of product bulk are processed to identify multispectral 11, 11', 11" images of single loose units 6 of product.

The process of identifying the multispectral 11, 11', 11" images of the single loose units 6 of product is carried out by means of an artificial neural network trained to segment the 10, 10', 10" images of the product mass in the container 5.

The trained neural network, particularly a deep neural network, performs image segmentation of the product mass by recognising the type of fruit and the images of single loose units of product, which are then characterised in shape and size.

Moreover, thanks to machine learning, the neural network is able to improve its capabilities and performance over time.

Finally, the multispectral 11, 11', 11" images of single loose units 6 of product are processed, e.g. with the aid of a computer, in order to classify the single loose units 6 of product with quality indices associated with their spectral response acquired by the multispectral sensor means 3.

The quality indices include at least one maturity index of the individual bulk unit of product.

The quality indices also include at least one hardness index of the single loose units of product.

The method provided by the present invention is very well suited to the internal and external inspection of very delicate small fruits and berries, as it involves a static investigation of the stationary product that does not require mechanical agitation.

The method provided by the present invention makes it possible to sample a batch of product without deteriorating the sampled product.

The quality inspection method for a fruit and vegetable product thus conceived is susceptible to numerous modifications and variations, all of which fall within the scope of the inventive concept according to the Claims.

## Claims

1. Quality inspection method of inspecting small fruits and berries product, comprising the following steps:
- providing optical reconstruction means including multispectral illuminating means (2) and multispectral sensor means (3);
- gathering loose units (6) of said product in a container (5), said container (5) being transparent of the basket type with an open top;
- illuminating with the multispectral illuminating means (2) the mass of said product present in said container (5) by emitting a sequence of light radiations with different bandwidths, said container (5) being illuminated from above;
- acquiring with said multispectral sensor means (3) multispectral images (10', 10") of said product mass present in said container (5) thus illuminated ;
- identifying multispectral images (11, 11', 11") of single loose units (6) of said product by processing said multispectral images (10, 10', 10") of said product mass present in said container (5);
- classifying said single loose units (6) with quality indices associated with the spectrum of their multispectral images (11, 11', 11") thus identified;
where the identification of said multispectral images (11, 11', 11") of said single loose units (6) of product is achieved by means of an artificial neural network trained to segment said images (10, 10', 10") of said product mass present in said container (5),
where the segmentation highlights the contours of said single loose units (6) of product that form the upper layer of said product mass.

2. Quality inspection method of inspecting small fruits and berries product according to the preceding claim, **characterised in that** said optical reconstruction means are configured for chlorophyll fluorescence imaging.

3. Quality inspection method of inspecting small fruits and berries product according to any preceding claim, **characterised in that** said optical reconstruction means are configured for imaging in a plurality of selected bands in the visible and near-infrared spectrum.

4. Quality inspection method of inspecting small fruits and berries product according to any preceding claim, **characterised by** the fact that said optical reconstruction means have selectable filters for band acquisition.

5. Quality inspection method of inspecting small fruits and berries product according to any preceding claim, **characterised by** the fact that said product includes cherries, strawberries, blueberries, blackberries, raspberries, currants, grapes.

6. Quality inspection method of inspecting small fruits and berries product according to any preceding claim, **characterised by** the fact that said acquisition with said multispectral sensor means (3) is carried out statically with said container (5) stationary at a station (4).

7. Quality inspection method of inspecting small fruits and berries product according to any preceding claim, **characterised in that** said station (4) is positioned in correspondence with a base (7a) of an obscurable chamber (7), and said multispectral sensor means (3) and said multispectral illuminating means (2) are positioned in correspondence with a ceiling (7b) of said obscurable chamber (7).

8. Quality inspection method of inspecting small fruits and berries product according to any one of claims 6 and 7, **characterised in that** said container (5) is supported by an indexed conveyor belt (9) configured for stationing said container (5) at said station (4).

9. Quality inspection method of inspecting small fruits and berries product according to any preceding claim, **characterised by** the fact that said quality indices include a ripeness index of said single loose unit (6) of product.

10. Quality inspection method of inspecting small fruits and berries product according to any preceding claim, **characterised by** the fact that said quality indices include a hardness index of said single loose unit (6) of product.

## Patentansprüche

1. Qualitätsprüfverfahren zur Inspektion von Kleinobst- und Beerenprodukten, umfassend die folgenden Schritte:
- Bereitstellen optischer Rekonstruktionsmittel, die multispektrale Beleuchtungsmittel (2) und multispektrale Sensormittel (3) umfassen;
- Sammeln loser Einheiten (6) des genannten Produkts in einem Behälter (5), wobei der Behälter (5) transparent ist, vom Korbtyp, mit offenem oberen Ende;
- Beleuchten der im Behälter (5) befindlichen Produktmasse mit den multispektralen Beleuchtungsmitteln (2) durch Aussenden einer Sequenz von Lichtstrahlungen mit unterschiedlichen Bandbreiten, wobei der Behälter (5) von oben beleuchtet wird;
- Erfassen multispektraler Bilder (10', 10") der so beleuchteten, im Behälter (5) befindlichen Produktmasse mit den multispektralen Sensormitteln (3);
- Identifizieren multispektraler Bilder (11, 11', 11") einzelner loser Einheiten (6) des genannten Produkts durch Verarbeitung der multispektralen Bilder (10, 10', 10") der im Behälter (5) befindlichen Produktmasse;
- Klassifizieren der einzelnen losen Einheiten (6) mit Qualitätsindizes, die dem Spektrum ihrer identifizierten multispektralen Bilder (11, 11', 11") zugeordnet sind;
wobei die Identifizierung der multispektralen Bilder (11, 11', 11") der einzelnen losen Produkteinheiten (6) mittels eines künstlichen neuronalen Netzes erfolgt, das zum Segmentieren der Bilder (10, 10', 10") der im Behälter (5) befindlichen Produktmasse trainiert ist,
wobei die Segmentierung die Konturen der einzelnen losen Produkteinheiten (6) hervorhebt, die die obere Schicht der Produktmasse bilden.

2. Qualitätsprüfverfahren nach dem vorhergehenden Anspruch, **dadurch gekennzeichnet, dass** die optischen Rekonstruktionsmittel für die Bildgebung mittels Chlorophyllfluoreszenz ausgebildet sind.

3. Qualitätsprüfverfahren nach einem der vorhergehenden Ansprüche, **dadurch gekennzeichnet, dass** die optischen Rekonstruktionsmittel für die Bildgebung in einer Mehrzahl ausgewählter Bänder im sichtbaren und nahinfraroten Spektralbereich ausgebildet sind.

4. Qualitätsprüfverfahren nach einem der vorhergehenden Ansprüche, **dadurch gekennzeichnet, dass** die optischen Rekonstruktionsmittel auswählbare Filter zur Bandaufnahme aufweisen.

5. Qualitätsprüfverfahren nach einem der vorhergehenden Ansprüche, **dadurch gekennzeichnet, dass** das Produkt Kirschen, Erdbeeren, Heidelbeeren, Brombeeren, Himbeeren, Johannisbeeren oder Trauben umfasst.

6. Qualitätsprüfverfahren nach einem der vorhergehenden Ansprüche, **dadurch gekennzeichnet, dass** die Erfassung mit den multispektralen Sensormitteln (3) statisch erfolgt, wobei der Behälter (5) an einer Station (4) stillsteht.

7. Qualitätsprüfverfahren nach einem der vorhergehenden Ansprüche, **dadurch gekennzeichnet, dass** die Station (4) in Zuordnung zu einem Boden (7a) einer abdunkelbaren Kammer (7) angeordnet ist und dass die multispektralen Sensormittel (3) sowie die multispektralen Beleuchtungsmittel (2) in Zuordnung zu einer Decke (7b) der abdunkelbaren Kammer (7) angeordnet sind.

8. Qualitätsprüfverfahren nach einem der Ansprüche 6 und 7, **dadurch gekennzeichnet, dass** der Behälter (5) von einem indexierten Förderband (9) getragen wird, das zum Positionieren des Behälters (5) an der Station (4) ausgebildet ist.

9. Qualitätsprüfverfahren nach einem der vorhergehenden Ansprüche, **dadurch gekennzeichnet, dass** die Qualitätsindizes einen Reifeindex der einzelnen losen Produkteinheit (6) umfassen.

10. Qualitätsprüfverfahren nach einem der vorhergehenden Ansprüche, **dadurch gekennzeichnet, dass** die Qualitätsindizes einen Härteindex der einzelnen losen Produkteinheit (6) umfassen.

## Revendications

1. Procédé d'inspection de la qualité destiné à l'inspection de produits constitués de petits fruits et de baies, comprenant les étapes suivantes :
- fournir des moyens de reconstruction optique comprenant des moyens d'éclairage multispectral (2) et des moyens de capteur multispectral (3) ;
- rassembler des unités libres (6) dudit produit dans un récipient (5), ledit récipient (5) étant transparent, de type panier, avec une partie supérieure ouverte ;
- éclairer, au moyen des moyens d'éclairage multispectral (2), la masse dudit produit présente dans ledit récipient (5) en émettant une séquence de radiations lumineuses ayant différentes largeurs de bande, ledit récipient (5) étant éclairé par le haut ;
- acquérir, au moyen desdits moyens de capteur multispectral (3), des images multispectrales (10', 10") de ladite masse de produit présente dans ledit récipient (5) ainsi éclairée ;
- identifier des images multispectrales (11, 11', 11") d'unités libres individuelles (6) dudit produit en traitant les images multispectrales (10, 10', 10") de ladite masse de produit présente dans ledit récipient (5) ;
- classifier lesdites unités libres individuelles (6) à l'aide d'indices de qualité associés au spectre de leurs images multispectrales (11, 11', 11") ainsi identifiées,
dans lequel l'identification desdites images multispectrales (11, 11', 11") desdites unités libres individuelles (6) de produit est réalisée au moyen d'un réseau neuronal artificiel entraîné à segmenter lesdites images (10, 10', 10") de ladite masse de produit présente dans ledit récipient (5), la segmentation mettant en évidence les contours desdites unités libres individuelles (6) de produit qui forment la couche supérieure de ladite masse de produit.

2. Procédé d'inspection de la qualité selon la revendication précédente, **caractérisé en ce que** lesdits moyens de reconstruction optique sont configurés pour l'imagerie par fluorescence de la chlorophylle.

3. Procédé d'inspection de la qualité selon l'une quelconque des revendications précédentes, **caractérisé en ce que** lesdits moyens de reconstruction optique sont configurés pour l'imagerie dans une pluralité de bandes sélectionnées du spectre visible et proche infrarouge.

4. Procédé d'inspection de la qualité selon l'une quelconque des revendications précédentes, **caractérisé par le fait que** lesdits moyens de reconstruction optique comportent des filtres sélectionnables pour l'acquisition de bandes.

5. Procédé d'inspection de la qualité selon l'une quelconque des revendications précédentes, **caractérisé par le fait que** ledit produit comprend des cerises, des fraises, des myrtilles, des mûres, des framboises, des groseilles et des raisins.

6. Procédé d'inspection de la qualité selon l'une quelconque des revendications précédentes, **caractérisé par le fait que** l'acquisition au moyen desdits moyens de capteur multispectral (3) est réalisée de manière statique, ledit récipient (5) étant immobile à une station (4).

7. Procédé d'inspection de la qualité selon l'une quelconque des revendications précédentes, **caractérisé en ce que** ladite station (4) est disposée au niveau d'une base (7a) d'une chambre occultable (7), et **en ce que** lesdits moyens de capteur multispectral (3) et lesdits moyens d'éclairage multispectral (2) sont disposés au niveau d'un plafond (7b) de ladite chambre occultable (7).

8. Procédé d'inspection de la qualité selon l'une quelconque des revendications 6 et 7, **caractérisé en ce que** ledit récipient (5) est supporté par un convoyeur indexé (9) configuré pour positionner ledit récipient (5) à ladite station (4).

9. Procédé d'inspection de la qualité selon l'une quelconque des revendications précédentes, **caractérisé par le fait que** lesdits indices de qualité comprennent un indice de maturité de ladite unité libre individuelle (6) de produit.

10. Procédé d'inspection de la qualité selon l'une quelconque des revendications précédentes, **caractérisé par le fait que** lesdits indices de qualité comprennent un indice de dureté de ladite unité libre individuelle (6) de produit.
